(19) Europäisches Patentamt
European Patent Office
Office européen des brevets

(11) Veröffentlichungsnummer: **0 351 617 B1**

# (12) EUROPÄISCHE PATENTSCHRIFT

(45) Veröffentlichungstag der Patentschrift: **28.09.94**

(51) Int. Cl.[5]: **C07D 213/647**, C07D 239/34, C07D 239/60, C07D 241/18, A01N 53/00

(21) Anmeldenummer: **89112040.4**

(22) Anmeldetag: **01.07.89**

Die Akte enthält technische Angaben, die nach dem Eingang der Anmeldung eingereicht wurden und die nicht in dieser Patentschrift enthalten sind.

## (54) Cyclopropancarboxamide, Verfahren zu ihrer Herstellung und ihre Verwendung zur Bekämpfung von Schädlingen.

(30) Priorität: **21.07.88 DE 3824788**

(43) Veröffentlichungstag der Anmeldung:
**24.01.90 Patentblatt 90/04**

(45) Bekanntmachung des Hinweises auf die Patenterteilung:
**28.09.94 Patentblatt 94/39**

(84) Benannte Vertragsstaaten:
**BE CH DE ES FR GB IT LI NL**

(56) Entgegenhaltungen:
**DE-A- 2 501 648**
**DE-A- 3 320 534**
**DE-A- 3 628 082**

(73) Patentinhaber: **BASF Aktiengesellschaft**
**Carl-Bosch-Strasse 38**
**D-67063 Ludwigshafen (DE)**

(72) Erfinder: **Kardorff, Uwe, Dr.**
**D 3,4**
**D-6800 Mannheim 1 (DE)**
Erfinder: **Neubauer, Hans-Juergen, Dr.**
**Mozartstrasse 6**
**D-4400 Muenster-Hiltrup (DE)**
Erfinder: **Leyendecker, Joachim, Dr.**
**Stahlbuehlring 79**
**D-6802 Ladenburg (DE)**
Erfinder: **Kuenast, Christoph, Dr.**
**Salierstrasse 2**
**D-6701 Otterstadt (DE)**
Erfinder: **Hofmeister, Peter, Dr.**
**Bernard-Humblot-Strasse 12**
**D-6730 Neustadt (DE)**
Erfinder: **Krieg, Wolfgang, Dr.**
**Saarstrasse 17**
**D-6721 Weingarten (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue Cyclopropancarboxamide der allgemeinen Formel I

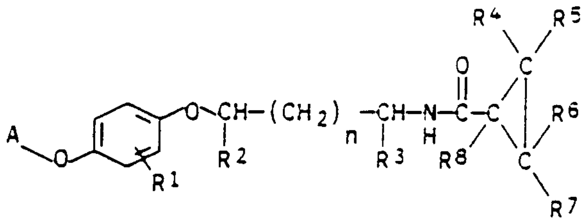

(I),

in der die Substituenten folgende Bedeutung haben:

| | |
|---|---|
| $R^1$ | Wasserstoff, Halogen, $C_1$-$C_3$-Alkyl, |
| $R^2$, $R^3$ | Wasserstoff, $C_1$-$C_4$-Alkyl, |
| $R^4$ -$R^8$ | Wasserstoff, Halogen, $C_1$-$C_4$-Alkyl, |
| A | ggf. durch $R^9$ substituiertes Hetaryl mit 1, 2 oder 3 Stickstoffatomen und sechs Ringgliedern, |
| $R^9$ | Halogen, $C_1$-$C_4$-Alkyl, $C_1$-$C_3$-Halogenalkyl, $C_1$-$C_4$-Alkoxy, $C_1$-$C_3$-Halogenalkoxy, $C_3$-$C_6$-Cycloalkyl, Cyano oder Nitro und |
| n | für 0 oder 1 steht. |

Außerdem betrifft die vorliegende Erfindung die Herstellung der Verbindungen I, Schädlingsbekämpfungsmittel, die die Verbindungen I enthalten, und ein Verfahren zur Bekämpfung von Schädlingen.

Aus der DE-A 33 20 534 ist der Carbaminsäureester A

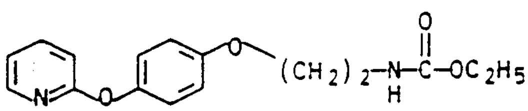

(A)

und aus der DE-A 36 28 082 (entspricht EP-A 258 733) das Cyclopropancarboxamid B

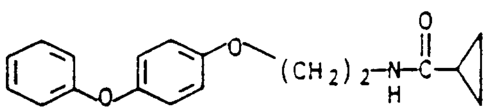

(B)

als Schädlingsbekämpfungsmittel bekannt.

Die Wirkung, Aufwandmenge und/oder Freilandstabilität der Verbindungen A und B sind jedoch nicht immer befriedigend.

Der Erfindung lag daher die Aufgabe zugrunde, neue Cyclopropancarboxamide I mit verbesserter und/oder gegen andere Schädlinge wirksamere Verbindungen I bereitzustellen.

Demgemäß wurden die eingangs definierten neuen Cyclopropancarboxamide I sowie Verfahren zu deren Herstellung gefunden. Ferner wurde gefunden, daß sich die Verbindungen I hervorragend als Schädlingsbekämpfungsmittel eignen.

Die Verbindungen I sind durch Umsetzung eines primären Amins der allgemeinen Formel II

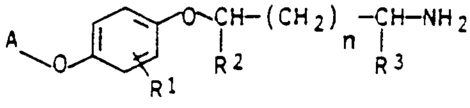

(II),

mit einem Cyclopropancarbonsäurehalogenid der allgemeinen Formel III

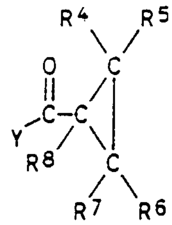

(III)

in der Y für Halogen steht, in Gegenwart eines Säureakzeptors erhältlich.

Als säurebindendes Mittel kann das Amin II eingesetzt werden. In der Regel verwendet man jedoch einen an sich üblichen Säureakzeptor wie aliphatische aromatische oder heterocyclische Amine z.B. Triethylamin, Dimethylamin, Diisopropylamin, Piperidin, Dimethylanilin, Dimethylbenzylamin, Pyridin und 4-Dimethylaminopyridin, wie Hydroxide von Alkali- und Erdalkalimetallen, z.B. Natriumhydroxid, Kaliumhydroxid, Calciumhydroxid, wie Alkoholate von Alkali- und Erdalkalimetallen z.B. Natriummethylat, Natriumethylat, Calciummethanolat, Kalium-tert.-butylat, wie Alkali- oder Erdalkalimetallhydride, z.B. Natriumhydrid, Kaliumhydrid oder Calciumhydrid, wie Alkali- oder Erdalkalicarbonate, z.B. Natriumcarbonat, Kaliumcarbonat oder Calciumcarbonat.

Die Umsetzungen werden zweckmäßigerweise in einem Lösungs- oder Verdünnungsmittel ausgeführt. Hierzu eignen sich aliphatische, aromatische, gegebenenfalls chlorierte Kohlenwasserstoffe wie beispielsweise Petrolether, n-Pentan, n-Hexan, Hexan-Isomerengemische, Benzol, Toluol, Xylol, Benzin, Dichlormethan, Chloroform, Tetrachlorkohlenstoff, 1,2-Dichlorethan und Chlorbenzol; Ether und Ester wie Diethyl- und Di-n-butylether, Methyl-tert.-butylether, Tetrahydrofuran, Dioxan, Essigsäureethylester; Ketone wie Aceton, Methylethylketon, Methylisopropylketon; Nitrile wie Aceto- und Propionitril; Alkohole wie Methanol, Ethanol, n-Propanol, Isopropanol; aprotische dipolare Lösungsmittel wie Dimethylformamid, Dimethylsulfoxid, Pyridin. Auch Gemische dieser Stoffe können als Lösungs- und Verdünnungsmittel verwendet werden.

Üblicherweise setzt man die Ausgangsstoffe II und III im äquimolaren Verhältnis ein, ein Überschuß der einen oder anderen Komponente kann in Einzelfällen aber durchaus vorteilhaft sein. Die Basenmenge liegt im allgemeinen bei 0,5 bis 20, vorzugsweise 0,7 bis 5, besonders bevorzugt bei 0,9 bis 1,5 mol pro Mol Säurehalogenid III.

Die Umsetzung kann bei Temperaturen von -30 bis 120 °C, vorzugsweise - 10 bis 80 °C, besonders bevorzugt 0 bis 50 °C, bei Normaldruck, über- oder Unterdruck nach den dafür üblichen Techniken vorgenommen werden.

Die zur Herstellung der Verbindungen I verwendeten primären Amine II sind teilweise literaturbekannt und können analog zu den entsprechenden Vorschriften, die in Houben-Weyl, Bd. VI, 3, Methoden der organischen Chemie, Thieme Verlag, 1965, 85 ff.,beschrieben sind, dargestellt werden.

Die außerdem benötigten Säurehalogenide, insbesondere Säurebromide und Säurechloride III sind bekannt oder in an sich bekannter Weise zugänglich und teilweise handelsüblich.

Die erfindungsgemäßen Verbindungen der Formel I können außerdem noch nach praktisch allen bekannten Verfahren der Carboxamidsynthese dargestellt werden, beispielsweise durch Umsetzung der entsprechenden primären Amine II mit entsprechenden Cyclopropancarbonsäureestern, Cyclopropancarbonsäuren oder deren Salzen, Cyclopropancarbonsäureanhydriden oder Ketenderivaten (vgl. C. Ferri, Reaktionen der organischen Synthese Georg Thieme Verlag, Stuttgart, 1978, S. 542 und dort zitierte Literatur).

Die neuen Verbindungen der Formel I fallen teilweise in Form farbloser oder schwach bräunlich gefärbter Öle an, die sich durch längeres Erwärmen unter vermindertem Druck auf mäßig erhöhte Temperaturen ("Andestillieren") von den letzten flüchtigen Anteilen befreien und auf diese Weise reinigen lassen. Erhält man die Verbindungen der Formel I in kristalliner Form, so kann ihre Reinigung durch Umkristallisation erfolgen.

In den Verbindungen der Formel I stehen die Reste $R^1$-$R^9$ vorzugsweise für folgende Substituenten:

$R^1$ für Wasserstoff;

Halogen wie Fluor, Chlor und Brom, bevorzugt Fluor und Chlor;

$C_1$-$C_3$ unverzweigtes oder verzweigtes Alkyl wie Methyl, Ethyl und Isopropyl;

$R^2$ und $R^3$ gleich oder verschieden für Wasserstoff;

unverzweigtes oder verzweigtes $C_1$-$C_4$-Alkyl wie Methyl, Ethyl, Propyl, Isopropyl, Butyl und isomeres Butyl, bevorzugt für Methyl und Ethyl oder für verzweigtes Alkyl, wie Isopropyl, Isobutyl und sec. Butyl;

$R^4$-$R^8$ gleich oder verschieden für Wasserstoff;
Halogen wie Fluor, Chlor und Brom, bevorzugt Fluor und Chlor;
unverzweigtes oder verzweigtes $C_1$-$C_4$-Alkyl wie Methyl, Ethyl, Propyl, Isopropyl, Butyl, bevorzugt Methyl, Ethyl und Isopropyl.

A für

- einen gegebenenfalls durch $R^9$ substituierten, stickstoffhaltigen Hetarylrest mit sechs Ringgliedern der allgemeinen Formeln IV.1-IV.5 steht,

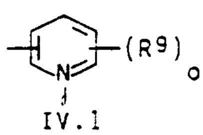 ,
IV.1

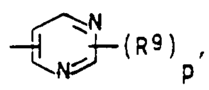 ,
IV.2

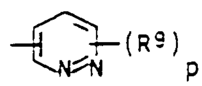 ,
IV.3

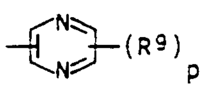 ,
IV.4

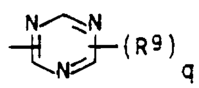 ,
IV.5

worin O eine Zahl 1 bis 4, p eine Zahl 1 bis 3 und q 1 oder 2 bedeutet und
$R^9$ für
Halogen wie Fluor, Chlor und Brom, bevorzugt Fluor und Chlor;
unverzweigtes oder verzweigtes $C_1$-$C_4$-Alkyl wie Methyl, Ethyl, Propyl, Butyl, bevorzugt für Methyl und Ethyl oder für verzweigtes Alkyl wie Isopropyl, Isobutyl und sec. Butyl;
unverzweigtes oder verzweigtes $C_1$-$C_3$-Halogenalkyl, bevorzugt Fluor- und Chloralkyl wie Fluormethyl, Difluormethyl, Trifluormethyl, Pentafluorethyl, 2,2,2-Trifluorethyl, Trichlormethyl und 2,2,2-Trichlorethyl;
unverzweigtes oder verzweigtes $C_1$-$C_4$-Alkoxy wie Methoxy, Ethoxy, Propoxy, Butoxy, bevorzugt Methoxy, Ethoxy und Isopropoxy;
unverzweigtes oder verzweigtes $C_1$-$C_3$-Halogenalkoxy, bevorzugt Fluor- und Chloralkoxy wie Trifluormethoxy, 1,1,2,2-Tetrafluorethoxy, Pentafluorethoxy oder Trichlormethoxy;
$C_3$-$C_6$-Cycloalkyl, wie Cyclopropyl, Cyclobutyl, Cyclopentyl, Cyclohexyl, wobei der Cyclopropylrest bevorzugt ist;
Cyano oder Nitro;
wobei im Fall von o, p oder q > 1 die Reste $R^9$ gleich oder verschieden sein können.

Besonders bevorzugte Hetarylreste sind:
Pyridin-2-yl, Pyridin-3-yl, Pyridin-4-yl, Pyrimidin-2-yl, Pyrimidin-4-yl, Pyrimidin-5-yl, Pyridazin-3-yl, Pyridazin-4-yl, Pyrazin-2-yl, Pyrazin-3-yl, s-Triazin-2-yl, besonders bevorzugt Pyridin-2-yl, Pyrimidin-2-yl, Pyrimidin-4-yl, Pyrimidin-5-yl, Pyridazin-3-yl und Pyrazin-3-yl.

Bevorzugt sind Cyclopropancarboxamide der Formel I, in der A unsubstituiertes oder durch $R^9$ substituiertes Pyridyl, Pyrimidyl, Pyridazinyl oder Pyrazinyl bedeutet.

Außerdem sind Cyclopropancarboxamide der Formel I bevorzugt, in der $R^1$, $R^3$ und $R^8$ Wasserstoff, $R^2$ Wasserstoff oder Methyl, $R^4$ bis $R^7$ Wasserstoff, Chlor, Fluor oder $C_1$-$C_4$-Alkyl und A unsubstituiertes oder durch $R^9$ substituiertes Pyrazinyl bedeuten und n für 0 steht.

Die erfindungsgemäßen Verbindungen I können ein oder mehrere Asymmetriezentren enthalten. Die vorliegende Erfindung schließt alle möglichen Stereoisomeren wie Diastereomeren, Enantiomeren sowie alle möglichen Diastereomeren- und Enantiomerengemische ein.

Die Cyclopropancarboxamide der allgemeinen Formel I sind geeignet, Schädlinge aus der Klasse der Insekten, Spinnentiere und Nematoden wirksam zu bekämpfen. Sie können im Pflanzenschutz sowie auf den Hygiene-, Vorratsschutz- und Veterinarsektor als Schädlingsbekämpfungsmittel eingesetzt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner),

Dendrolimus pini (Kiefernspinner), Thaumatopoea ptiyocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagai (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Rape-Flohkäfer), Diabrotica 12-puncta (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Liniierter Blattrandkäfer), Ortiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrynchus napi (Großer Kohltriebrüßler), Sitophilus granaria (Kornkäfer), Anisandrus dispar (Ungleicher Holzborkenkäfer), Ips typographus (Buchdrukker), Blastophagus piniperda (Gefurchter Waldgärtner);

aus der Ordnung der Zweiflügler (Diptera) beispielsweise Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);

aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilaparvata lugens (Braune Reis-Zikade), Empoasca fabae (Kartoffelzikade), Psylla mali (Apfelblattsauger), Psylla piri (Birnenblattsauger), Trialeurodes vaporariorum (Weiße Fliege Mottenschildlaus), Aphis fabae (Schwarze Bohnenlaus), Aphis pomi (Grüne Apfelblattlaus), Aphis sambuci (Holunderblattlaus), Aphidula nasturtii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehige Apfelfaltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenblattlaus), Macrosiphon rosae (Große Rosenblattlaus), Megoura viciae (Wickenblattlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), viteus vitifolii (Reblaus);

aus der Ordnung der Termiten (Isoptera) beispielsweise Reticulitermes lucifugus, Calotermes flavicollis, Leucotermes flavipes, Termes natalensis;

aus der Ordnung der Geradflügler (Orthoptera) beispielsweise Forficula auricularia (Gemeiner Ohrwurm),

Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschre ke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blabera gigantes (Riesenschabe).

Zur Klasse der Arachnoidea gehören Spinnentiere (Acarina) beispielsweise Ixodes ricinus (Holzbock), Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zystenbildende Nematoden, z.B. Globodera rostochiensis, Heterodera schachtii, Heterodera avenae, Hetrodera glycinae, Heterodera trifolii, Stock- und Blattälchen, z.B. Ditylenchus dipsaci, Ditylenchus destructor, Pratylenchus neglectus, Pratylenchus penetrans, Partylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Verfahren zur Bekämpfung der Schädlinge sind dadurch gekennzeichnet, daß man eine wirksame Menge eines Cyclopropancarboxamides der Formel I auf die Schädlinge bzw. deren Lebensraum einwirken läßt.

Die Wirkstoffe können als solche, in Form ihrer Formulierungen oder den daraus bereiteten Anwendungsmöglichkeiten, z.B. in Form direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Aklylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 6 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 6 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 1 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 1 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10, vorzugsweise zwischen 0,01 und 1 Gew.-%. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,01 bis 10, vorzugsweise 0,05 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Herstellungsbeispiel:

Beispiel 1:

N-(2-[4-(6-Chlorpyridin-2-yl-oxy)-phenoxy]-eth-1-yl]-cyclopropancarbonsäureamid

Zu 2,6 g 2-[4-(6-Chlorpyridin-2-yl-oxy)-phenoxy]-ethylamin in 50 $cm^3$ Dichlormethan werden bei Raumtemperatur nacheinander 1,1 g Triethylamin und 1,0 g Cyclopropancarbonsäurechlorid dazugetropft. Die Reaktion verläuft leicht exotherm. Bei Raumtemperatur läßt man 14 h rühren.

Dann wird auf Eiswasser gegossen und mit Dichlormethan extrahiert. Die vereinigten organischen Phasen werden über Natriumsulfat getrocknet und im Rotationsverdampfer eingeengt. Es verbleibt ein weißer Feststoff, Schmelzbereich 114-117 °C.

Ausbeute:

1,0 g ≙ 30,6 % der rechnerisch möglichen Menge.

Entsprechend zu diesem Darstellungsbeispiel können die in der folgenden Tabelle 1 beschriebenen Verbindungen I hergestellt werden. Im Falle nicht kristalliner Substanzen werden die intensivsten Infrarotabsorptionen im Bereich kleiner ungefähr 1500 $cm^{-1}$ angegeben.

Tabelle 1:

Cyclopropancarboxamide

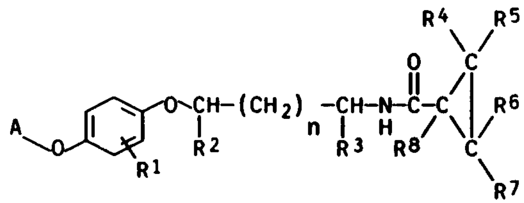 (I),

| Bsp.-Nr. | A | A gebunden in ...-Stellung | $R^1$ | $R^2$ | $R^3$ | $R^4$ | $R^5$ | $R^6$ | $R^7$ | $R^8$ | n | Schmp ($^oC$) bzw. bzw. IR-Daten ($cm^{-1}$) |
|---|---|---|---|---|---|---|---|---|---|---|---|---|
| 1 | 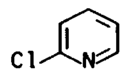 | 2 | H | H | H | H | H | H | H | H | 0 | 114-117 |
| 2 | 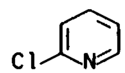 | 2 | H | $CH_3$ | H | H | H | H | H | H | 0 | |
| 3 | 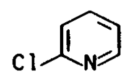 | 2 | H | H | H | $CH_3$ | H | H | H | H | 0 | |
| 4 | 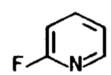 | 2 | H | H | H | H | H | H | H | H | 0 | 74-77 |
| 5 | 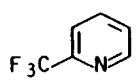 | 2 | H | H | H | $CH_3$ | H | H | H | H | 0 | |
| 6 | 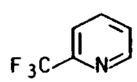 | 2 | H | H | H | H | H | H | H | H | 0 | 116-118 |

Tabelle 1 Forts.

| Bsp.-Nr. | A | A gebunden in ...-Stellung | $R^1$ | $R^2$ | $R^3$ | $R^4$ | $R^5$ | $R^6$ | $R^7$ | $R^8$ | n | Schmp ($^oC$) bzw. bzw. IR-Daten ($cm^{-1}$) |
|---|---|---|---|---|---|---|---|---|---|---|---|---|
| 7 | 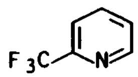 | 2 | H | H | H | $CH_3$ | H | H | H | H | 0 | |
| 8 | 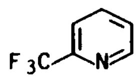 | 2 | H | H | H | Cl | Cl | $CH_3$ | $CH_3$ | H | 0 | |
| 9 | 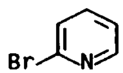 | 2 | H | H | H | H | H | H | H | H | 0 | 123-128 |
| 10 | 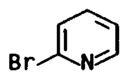 | 2 | H | H | H | $CH_3$ | H | H | H | H | 0 | |
| 11 | 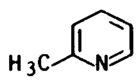 | 2 | H | H | H | H | H | H | H | H | 0 | 100-105 |
| 12 | 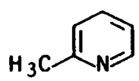 | 2 | H | H | H | $CH_3$ | H | H | H | H | 0 | |
| 13 | 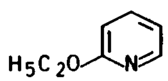 | 2 | H | H | H | H | H | H | H | H | 0 | |
| 14 | 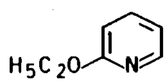 | 2 | H | H | H | $CH_3$ | H | H | H | H | 0 | |
| 15 | 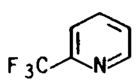 | 2 | H | $CH_3$ | H | H | H | H | H | H | 0 | |

Tabelle 1 Forts.

| Bsp.-Nr. | A | A gebunden in ...-Stellung | $R^1$ | $R^2$ | $R^3$ | $R^4$ | $R^5$ | $R^6$ | $R^7$ | $R^8$ | n | Schmp ($^oC$) bzw. bzw. IR-Daten ($cm^{-1}$) |
|---|---|---|---|---|---|---|---|---|---|---|---|---|
| 16 | 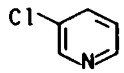 | 2 | H | H | H | H | H | H | H | H | 0 | 111-114 |
| 17 | 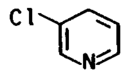 | 2 | H | H | H | $CH_3$ | H | H | H | H | 0 | |
| 18 | 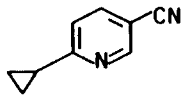 | 2 | H | H | H | H | H | H | H | H | 0 | |
| 19 | 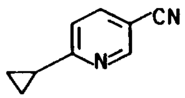 | 2 | H | H | H | $CH_3$ | H | H | H | H | 0 | |
| 20 | 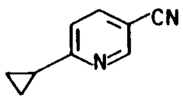 | 2 | H | $CH_3$ | H | H | H | H | H | H | 0 | |
| 21 | 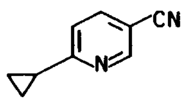 | 2 | H | $CH_3$ | H | $CH_3$ | H | H | H | H | 0 | |
| 22 | 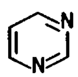 | 2 | H | H | H | H | H | H | H | H | 0 | 124 |

Tabelle 1 Forts.

| Bsp.-Nr. | A | A gebunden in ...-Stellung | $R^1$ | $R^2$ | $R^3$ | $R^4$ | $R^5$ | $R^6$ | $R^7$ | $R^8$ | n | Schmp (°C) bzw. bzw. IR-Daten ($cm^{-1}$) |
|---|---|---|---|---|---|---|---|---|---|---|---|---|
| 23 | 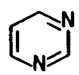 | 2 | H | H | H | $CH_3$ | H | H | H | H | 0 | 101 |
| 24 | 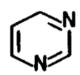 | 2 | H | $CH_3$ | H | H | H | H | H | H | 0 | 113 |
| 25 | 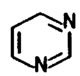 | 2 | H | $CH_3$ | H | $CH_3$ | H | H | H | H | 0 | 114-115 |
| 26 | 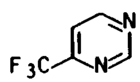 | 2 | H | H | H | H | H | H | H | H | 0 | |
| 27 | 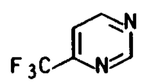 | 2 | H | H | H | $CH_3$ | H | H | H | H | 0 | |
| 28 | 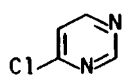 | 2 | H | H | H | H | H | H | H | H | 0 | 103 |
| 29 | 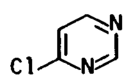 | 2 | H | H | H | $CH_3$ | H | H | H | H | 0 | 99-100 |
| 30 | 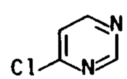 | 2 | H | $CH_3$ | H | H | H | H | H | H | 0 | 143 |
| 31 | 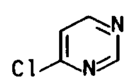 | 2 | H | $CH_3$ | H | $CH_3$ | H | H | H | H | 0 | |

Tabelle 1 Forts.

| Bsp.-Nr. | A | A gebunden in ...-Stellung | $R^1$ | $R^2$ | $R^3$ | $R^4$ | $R^5$ | $R^6$ | $R^7$ | $R^8$ | n | Schmp (°C) bzw. bzw. IR-Daten ($cm^{-1}$) |
|---|---|---|---|---|---|---|---|---|---|---|---|---|
| 32 | 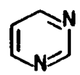 | 5 | H | H | H | H | H | H | H | H | 0 | 98 |
| 33 | 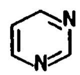 | 5 | H | H | H | $CH_3$ | H | H | H | H | 0 | 99-100 |
| 34 | 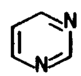 | 5 | H | $CH_3$ | H | H | H | H | H | H | 0 | |
| 35 | 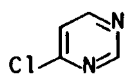 | 5 | H | $CH_3$ | H | $CH_3$ | H | H | H | H | 0 | |
| 36 | 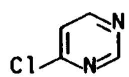 | 4 | H | H | H | H | H | H | H | H | 0 | |
| 37 | 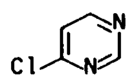 | 4 | H | H | H | $CH_3$ | H | H | H | H | 0 | |
| 38 | 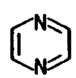 | 2 | H | H | H | H | H | H | H | H | 0 | 121-126 |
| 39 | 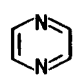 | 2 | H | H | H | $CH_3$ | H | H | H | H | 0 | 98-101 |

Tabelle 1 Forts.

| Bsp.-Nr. | A | A gebunden in ...-Stellung | $R^1$ | $R^2$ | $R^3$ | $R^4$ | $R^5$ | $R^6$ | $R^7$ | $R^8$ | n | Schmp (°C) bzw. bzw. IR-Daten ($cm^{-1}$) |
|---|---|---|---|---|---|---|---|---|---|---|---|---|
| 40 | 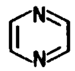 | 2 | H | $CH_3$ | H | H | H | H | H | H | 0 | 103-107 |
| 41 | 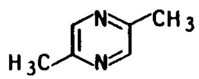 | 2 | H | H | H | H | H | H | H | H | 0 | 108-112 |
| 42 | 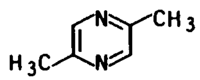 | 2 | H | H | H | $CH_3$ | H | H | H | H | 0 | 120-122 |
| 43 | 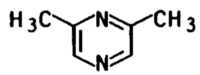 | 2 | H | H | H | H | H | H | H | H | 0 | |
| 44 | 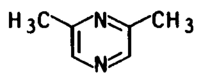 | 2 | H | H | H | $CH_3$ | H | H | H | H | 0 | 90-94 |
| 45 | 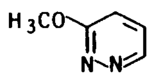 | 3 | H | H | H | H | H | H | H | H | 0 | |
| 46 | 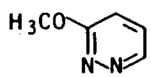 | 3 | H | H | H | $CH_3$ | H | H | H | H | 0 | |
| 47 | 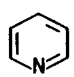 | 2 | H | H | H | H | H | H | H | H | 0 | 60-64 |

Tabelle 1 Forts.

| Bsp.-Nr. | A | A gebunden in ...-Stellung | $R^1$ | $R^2$ | $R^3$ | $R^4$ | $R^5$ | $R^6$ | $R^7$ | $R^8$ | n | Schmp (°C) bzw. bzw. IR-Daten ($cm^{-1}$) |
|---|---|---|---|---|---|---|---|---|---|---|---|---|
| 48 | 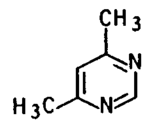 | 2 | H | H | H | H | H | H | H | H | 0 | 162 |
| 49 | 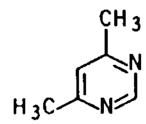 | 2 | H | H | H | $CH_3$ | H | H | H | H | 0 | 128 |
| 50 | 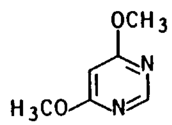 | 2 | H | H | H | H | H | H | H | H | 0 | 137-138 |
| 51 | 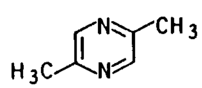 | 2 | H | $CH_3$ | H | H | H | H | H | H | 0 | 125-129 |
| 52 | 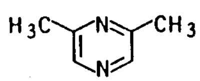 | 2 | H | $CH_3$ | H | H | H | H | H | H | 0 | 112-115 |
| 53 | 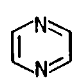 | 2 | H | $CH_3$ | H | $CH_3$ | H | H | H | H | 0 | 100-108 |
| 54 | 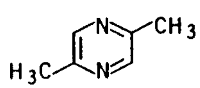 | 2 | H | $CH_3$ | H | $CH_3$ | H | H | H | H | 0 | 82-86 |

Anwendungsbeispiel

Im folgenden Beispiel wurde die erfindungsgemäße Verbindung aus Beispiel I bzw. sie enthaltende Mittel hinsichtlich ihrer Wirkung auf Schädlinge mit der nachstehenden Verbindung des Standes der

Technik verglichen. Die Reinheit der Substanz Nr. 1 wie die des Vergleichsmittels A lag bei > 95 %.

A: 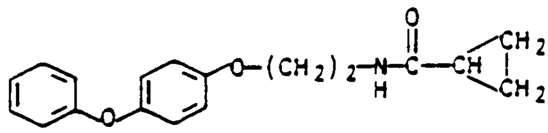

bekannt aus
EP-A-258 733
Beispiel 2

Die Konzentrationen, bei denen die untersuchten Verbindungen eine 100 %ige Mortalität bzw. Hemmung bewirken, sind die jeweiligen Minimalkonzentrationen. Bei jeder Konzentration wurden mindestens zwei Versuche angesetzt und ein Mittelwert gebildet.

Beispiel A

Dysdercus intermedius (Baumwollwanze), ovizide Wirkung

Der Versuch erfolgt in 1 1-Gläser auf 200 g sterilem Quarzsand (Korngröße 0-3 mm). Dieser Sand wird vor Versuchsbeginn mit 20 ml der wäßrigen Wirkstoffaufbereitung angegossen. Darauf belegt man die Gläser mit 10 Larven des vierten Larvenstadiums. Als Futter bietet man gequollene Baumwollsaat, die wöchentlich gewechselt wird. Ebenfalls wöchentlich wird der Sand mit reinem Wasser angefeuchtet. Die Versuchstemperatur beträgt 25 bis 27°. Die Beobachtungszeit erstreckt sich bis zur Adulthäutung. Die Probe gilt als Wirksam, wenn die Tiere bei Versuchsende entweder tot sind oder Riesenlarven oder Zwischentypen (Adultoide) darstellen oder stärkere morphologische Defekte aufweisen.

Die Versuchsergebnisse sind in Tabelle A zusammengefaßt.

Tabelle A

| Verbindung Nr. | Wirkstoffkonzentration [ppm] | Mortalität (%) |
|---|---|---|
| 1 | 0,1 | 100 |
| A | 1 | 100 |

**Patentansprüche**

**1.** Cyclopropancarboxamide der allgemeinen Formel I

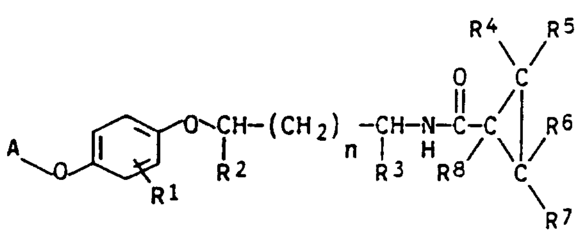 (I),

in der die Substituenten folgende Bedeutung haben:

| | |
|---|---|
| $R^1$ | Wasserstoff, Halogen, $C_1$-$C_3$-Alkyl, |
| $R^2$, $R^3$ | Wasserstoff, $C_1$-$C_4$-Alkyl, |
| $R^4$ - $R^8$ | Wasserstoff, Halogen, $C_1$-$C_4$-Alkyl, |
| A | ggf. durch $R^9$ substituiertes Hetaryl mit 1, 2 oder 3 Stickstoffatomen und sechs Ringgliedern, |
| $R^9$ | Halogen, $C_1$-$C_4$-Alkyl, $C_1$-$C_3$-Halogenalkyl, $C_1$-$C_4$-Alkoxy, $C_1$-$C_3$-Halogenalkoxy, $C_3$-$C_6$-Cycloalkyl, Cyano oder Nitro, und |

n für 0 oder 1 steht.

2. Cyclopropancarboxamide der Formel I gemäß Anspruch 1, in der A unsubstituiertes oder durch $R^9$ substituiertes Pyridyl, Pyrimidyl, Pyridazinyl oder Pyrazinyl bedeutet.

3. Cyclopropancarboxamide der Formel I gemäß Anspruch 1, in der
   - $R^1$, $R^3$ und $R^8$ Wasserstoff,
   - $R^2$ Wasserstoff oder Methyl,
   - $R^4$ - $R^7$ Wasserstoff, Chlor, Fluor oder $C_1$-$C_4$-Alkyl und
   - A unsubstituiertes oder durch $R^9$ substituiertes Pyridyl, Pyrimidyl, Pyridazinyl oder Pyrazinyl

   bedeuten und n für 0 steht.

4. Verfahren zur Herstellung von Cyclopropancarboxamiden der allgemeinen Formel I gemäß Anspruch 1, dadurch gekennzeichnet, daß man ein primäres Amin der allgemeinen Formel II

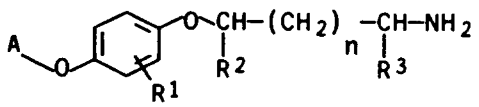

(II),

mit einem Cyclopropancarbonsäurehalogenid der allgemeinen Formel III

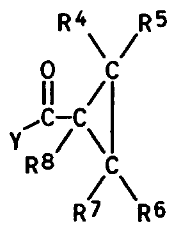

(III),

in der Y für Halogen steht, in Gegenwart eines Säureakzeptors umsetzt.

5. Schädlingsbekämpfungsmittel, enthaltend ein Cyclopropancarboxamid der Formel I gemäß Anspruch 1 neben hierfür üblichen Trägerstoffen.

6. Schädlingsbekämpfungsmitel gemäß Anspruch 5, dadurch gekennzeichnet, daß es 0,1 bis 95 Gew.-% eines Cyclopropancarboxamids der Formel I enthält.

7. Verfahren zur Bekämpfung von Schädlingen, dadurch gekennzeichnet, daß man eine wirksame Menge eines Cyclopropancarboxamides der Formel I gemäß Anspruch 1 auf die Schädlinge bzw. deren Lebensraum einwirken läßt.

## Claims

1. A cyclopropanecarboxamide of the general formula I

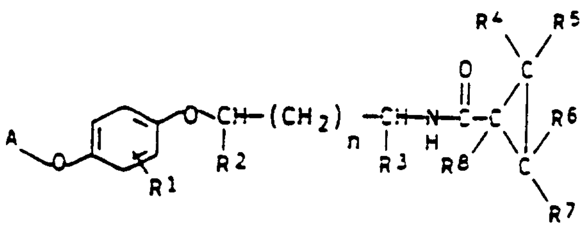

(I)

where $R^1$ is hydrogen, halogen or $C_1$-$C_3$-alkyl, $R^2$ and $R^3$ are each hydrogen or $C_1$-$C_4$-alkyl, $R^4$-$R^8$ are each hydrogen, halogen or $C_1$-$C_4$-alkyl, A is unsubstituted or $R^9$substituted hetaryl having 1, 2 or 3 nitrogen atoms and six ring members, $R^9$ is halogen, $C_1$-$C_4$-alkyl, $C_1$-$C_3$-haloalkyl, $C_1$-$C_4$-alkoxy, $C_1$-$C_3$-haloalkoxy, $C_3$-$C_6$-cycloalkyl, cyano or nitro, and n is 0 or 1.

2. A cyclopropanecarboxamide of the formula I as claimed in claim 1, where A is unsubstituted or R9-substituted pyridyl, pyrimidyl, pyridazinyl or pyrazinyl.

3. A cyclopropanecarboxamide of the formula I as claimed in claim 1, where $R^1$, $R^3$ and $R^8$ are each hydrogen, $R^2$ is hydrogen or methyl, $R^4$-$R^7$ are each hydrogen, chlorine, fluorine or $C_1$-$C_4$-alkyl, and A is unsubstituted or $R^9$-substituted pyridyl, pyrimidyl, pyridazinyl or pyrazinyl, and n is 0.

4. A process for the preparation of a cyclopropanecarboxamide of the general formula I as claimed in claim 1, which comprises reacting a primary amine of the general formula II

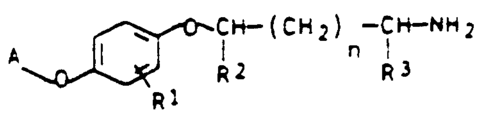

(II)

with a cyclopropanecarbonyl halide of the general formula III

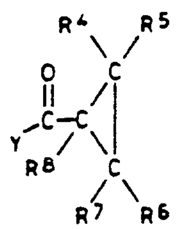

(III)

where Y is halogen, in the presence of an acid acceptor.

5. A pesticide containing a cyclopropanecarboxamide of the formula I as claimed in claim 1, together with conventional carriers.

6. A pesticide as claimed in claim 5, containing from 0.1 to 95 % by weight of a cyclopropanecarboxamide of the formula I.

7. A method for controlling pests, wherein an effective amount of a cyclopropanecarboxamide of the formula I as claimed in claim 1 is allowed to act on the pests or their habitat.

**Revendications**

1. Cyclopropanecarboxamides de formule générale I

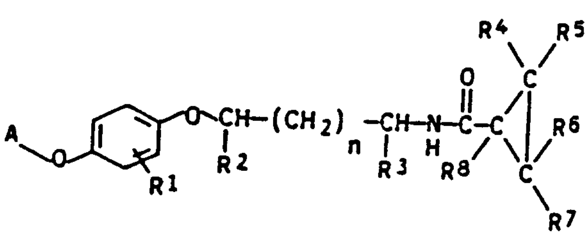

(I),

dans laquelle les substituants ont la signification suivante :

$R^1$ hydrogène, halogène, alkyle en C1-C3,

| | |
|---|---|
| $R^2$, $R^3$ | hydrogène, alkyle en C1-C4, |
| $R^4$-$R^8$ | hydrogène, halogène, alkyle en C1-C4, |
| A | hétaryle éventuellement substitué par $R^9$, à 1, 2 ou 3 atomes d'azote et six chaînons du cycle, |
| $R^9$ | halogène, alkyle en C1-C4, halogène, alkyle en C1-C4, halogène-alkyle en C1-C3, alcoxy en C1-C4, halogène-alcoxy en C1-C3, cycloalkyle en C3-C6, cyano ou nitro, |
| n | étant mis pour 0 ou 1. |

**2.** Cyclopropanecarboxamides de formule I, selon la revendication 1, dans laquelle A représente pyridyle, pyrimidyle, pyridazinyle ou pyrazinyle non substitués ou substitués par $R^9$.

**3.** Cyclopropanecarboxamides de formule I, selon la revendication 1, dans laquelle

| | |
|---|---|
| $R^1$, $R^3$ et $R^8$ | représentent hydrogène, |
| $R^2$ | représente hydrogène ou méthyle, |
| $R^4$-$R^7$ | représentent hydrogène, chlore, fluor ou alkyle en C1-C4 et |
| A | représente pyridyle, pyrimidyle, pyridazinyle ou pyrazinyle non substitués ou substitués par $R^9$, |
| n | étant mis pour 0. |

**4.** Procédé de préparation de cyclopropanecarboxamides de formule générale I, selon la revendication 1, caractérisé par le fait que l'on met à réagir, en présence d'un liant d'acide, une amine primaire de formule générale II

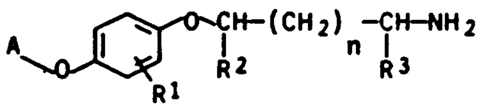

(II),

avec un halogènure d'acide cyclopropanecarboxylique de formule générale III

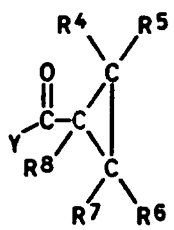

(III),

dans laquelle Y est mis pour halogène.

**5.** Pesticide selon la revendication 5, caractérisé par le fait qu'il contient un cyclopropanecarboxamide de formule I, selon la revendication 1, outre des supports respectifs usuels.

**6.** Pesticide selon la revendication 5, caractérisé par le fait qu'il contient 0,1 à 95 % en poids d'un cyclopropanecarboxamide de formule 1.

**7.** Procédé de lutte contre les parasites, caractérisé par le fait que l'on fait agir sur les parasites ou leur biotope une quantité efficace d'un cyclopropanecarboxamide de formule I, selon la revendication 1.